# EUROPEAN PATENT APPLICATION

(11) **EP 3 973 939 A1**
(43) Date of publication of application: **30.03.2022**
(21) Application number: 20809540.6
(22) Date of filing: 19.05.2020
(51) Int. Cl.: A61J 1/05, G01N 33/48

(54) **COMPOSITION FOR MONONUCLEAR CELL-CONTAINING PLASMA SEPARATION AND BLOOD SAMPLING CONTAINER**

(30) Priority: 20.05.2019 JP 2019094270
(71) Applicant: Sekisui Medical Co., Ltd., Tokyo 103-0027 (JP); Tokuyama Sekisui Co., Ltd., Osaka-Shi, Osaka 530-8565 (JP)
(72) Inventor: KOMAI, Kuniya, Shunan-shi, Yamaguchi 746-0006 (JP); ISOGAWA, Hironobu, Shunan-shi, Yamaguchi 746-0006 (JP); ANRAKU, Hideo, Shunan-shi, Yamaguchi 746-0006 (JP)
(74) Representative: Ter Meer Steinmeister & Partner
(86) International application number: PCT/JP2020/019727
(87) International publication number: WO 2020/235544

(57) **Abstract**

Provided is a composition for mononuclear cell-containing plasma separation capable of suppressing a flow of the composition for mononuclear cell-containing plasma separation during storage, capable of satisfactorily forming a partition during centrifugation, and capable of yielding mononuclear cell-containing plasma with little admixture of blood cell components other than mononuclear cells. The composition for mononuclear cell-containing plasma separation according to the present invention contains an organic component having fluidity at 25°C and two or more kinds of inorganic fine powders, and has a specific gravity of 1.060 or more and 1.080 or less at 25°C.

## Description

### TECHNICAL FIELD

The present invention relates to a composition for mononuclear cell-containing plasma separation used for separating a plasma containing mononuclear cells from blood. The present invention also relates to a blood sampling container with the composition for mononuclear cell-containing plasma separation contained in a blood sampling container main body.

### BACKGROUND ART

In clinical examinations, blood sampling containers are widely used to collect a blood sample. In a blood sampling container inside which a composition for serum or plasma separation is contained, the specific gravity of the composition for separation can be appropriately adjusted to separate serum or plasma from whole blood by centrifugation using the difference in specific gravity.

Patent Document 1 below discloses a composition for serum or plasma separation containing a liquid organic compound, a thixotropy-imparting component and a thermoplastic elastomer. In addition, in all Examples of Patent Document 1, compositions for serum or plasma separation having a specific gravity of 1.043 to 1.045 at 25°C are prepared. The composition for serum or plasma separation of Patent Document 1 has thixotropy, making it possible to, for example, prevent the composition for serum or plasma separation contained in the bottom of a blood sampling container main body from flowing to the open end side of the blood sampling container main body.

In addition, a composition for separation capable of separating a specific blood cell component from blood is also known.

Patent Document 2 below discloses a method for separating platelets, lymphocytes and mononuclear large leukocytes (monocytes) from anticoagulated blood, including the following steps (a), (b) and (c). (a) A water-insoluble thixotropic gel-like substance (composition for separation) that is chemically inactive with respect to blood components and has a specific gravity of 1.065 to 1.077 g/cc is put into an anticoagulated blood sample. (b) The gel-like substance and the blood sample are centrifuged at a force of at least 1200 G for a sufficient time for the gel-like substance to form a barrier between plasma, platelets, lymphocytes and mononuclear large leukocytes (monocytes), and heavier blood cells. (c) The plasma, platelets, lymphocytes and mononuclear large leukocytes on the barrier are collected. In addition, in Patent Document 2, silica powder is used as an inorganic fine powder contained in the water-insoluble thixotropic gel-like substance (composition for separation).

### Related Art Document

### Patent Document

Patent Document 1: WO 2016/199851 A1
Patent Document 2: JP H02-111374 A

### SUMMARY OF THE INVENTION

### PROBLEMS TO BE SOLVED BY THE INVENTION

In clinical examinations, tests using mononuclear cells (lymphocytes and monocytes) have been performed. However, mononuclear cell-using tests using a sample solution containing blood cell components other than mononuclear cells (for example, erythrocytes and granulocytes) may affect the test results.

Conventional compositions for plasma separation such as those described in Patent Document 1 are not intended to separate mononuclear cells from blood cell components other than the mononuclear cells. Accordingly, when plasma is separated using a conventional composition for plasma separation, the number of mononuclear cells contained in the obtained plasma is extremely small, making it difficult to use the plasma as a sample solution for mononuclear cell-using test.

On the other hand, the water-insoluble thixotropic gel-like substance (composition for separation) described in Patent Document 2 can separate platelets, lymphocytes and mononuclear large leukocytes (monocytes) from blood. However, when the specific gravity of the composition for separation is increased using only silica powder as an inorganic fine powder as described in Patent Document 2, the viscosity and thixotropic index of the composition for separation become too high for the composition for separation to exhibit a sufficient fluidity during centrifugation, making it difficult to satisfactorily form a partition. In Patent Document 2, although the formability of a partition is improved to some extent by placing a water-insoluble thixotropic gel-like substance (composition for separation) on the liquid surface of sampled blood after blood sampling, the operation of opening a blood sampling container after blood sampling and placing the composition for separation on the liquid surface of the blood is not practical in clinical examinations.

In addition, using a conventional composition for separation, the composition for separation may flow during storage. For example, using a conventional composition for separation, the composition for separation contained in the bottom of a blood sampling container main body may flow to the open end side of the blood sampling container main body. When the flow of the composition for separation occurs, a blood sampling needle may be blocked with the composition for separation at the time of blood collection, resulting in poor blood sampling.

An object of the present invention is to provide a composition for mononuclear cell-containing plasma separation capable of suppressing a flow of the composition for mononuclear cell-containing plasma separation during storage, capable of satisfactorily forming a partition during centrifugation, and capable of yielding mononuclear cell-containing plasma with little admixture of blood cell components other than mononuclear cells. Another object of the present invention is to provide a blood sampling container containing the composition for mononuclear cell-containing plasma separation.

### MEANS FOR SOLVING THE PROBLEMS

According to a broad aspect of the present invention, a composition for mononuclear cell-containing plasma separation is provided, including an organic component having fluidity at 25°C and two or more kinds of inorganic fine powders, and having a specific gravity of 1.060 or more and 1.080 or less at 25°C.

In a certain aspect of the composition for mononuclear cell-containing plasma separation according to the present invention, the organic component having fluidity at 25°C contains a resin.

In another certain aspect of the composition for mononuclear cell-containing plasma separation according to the present invention, the organic component having fluidity at 25°C is a mixture of the resin and an alkyl benzenepolycarboxylate ester derivative.

In yet another certain aspect of the composition for mononuclear cell-containing plasma separation according to the present invention, the resin includes a petroleum resin, a cyclopentadiene-based resin, or a polyester resin.

In yet another certain aspect of the composition for mononuclear cell-containing plasma separation according to the present invention, the resin includes a petroleum resin or a cyclopentadiene-based resin.

In yet another certain aspect of the composition for mononuclear cell-containing plasma separation according to the present invention, the inorganic fine powders include a fine powder silica.

In yet another certain aspect of the composition for mononuclear cell-containing plasma separation according to the present invention, the inorganic fine powders include a fine powder silica and an inorganic fine powder having a specific gravity of 3 or more.

In yet another certain aspect of the composition for mononuclear cell-containing plasma separation according to the present invention, the fine powder silica includes a hydrophilic silica.

In yet another certain aspect of the composition for mononuclear cell-containing plasma separation according to the present invention, the fine powder silica includes a hydrophilic silica and a hydrophobic silica.

In yet another certain aspect of the composition for mononuclear cell-containing plasma separation according to the present invention, the composition for mononuclear cell-containing plasma separation includes an organic gelling agent.

According to a broad aspect of the present invention, a blood sampling container is provided, including a blood sampling container main body and the composition for mononuclear cell-containing plasma separation, the composition for mononuclear cell-containing plasma separation being contained in the blood sampling container main body.

### EFFECT OF THE INVENTION

The composition for mononuclear cell-containing plasma separation according to the present invention contains an organic component having fluidity at 25°C and two or more kinds of inorganic fine powders, and has a specific gravity of 1.060 or more and 1.080 or less at 25°C. Since the composition for mononuclear cell-containing plasma separation according to the present invention has the above-mentioned structure, the composition for mononuclear cell-containing plasma separation is capable of suppressing a flow of the composition for mononuclear cell-containing plasma separation during storage, capable of satisfactorily forming a partition during centrifugation, and capable of yielding mononuclear cell-containing plasma with little admixture of blood cell components other than mononuclear cells.

### MODES FOR CARRYING OUT THE INVENTION

Hereinafter, a detailed description is made of the present invention.

The composition for mononuclear cell-containing plasma separation according to the present invention contains an organic component having fluidity at 25°C and two or more kinds of inorganic fine powders. The composition for mononuclear cell-containing plasma separation according to the present invention has a specific gravity of 1.060 or more and 1.080 or less at 25°C.

Since the composition for mononuclear cell-containing plasma separation according to the present invention has the above-mentioned structure, the composition for mononuclear cell-containing plasma separation is capable of suppressing a flow of the composition for mononuclear cell-containing plasma separation during storage, capable of satisfactorily forming a partition during centrifugation, and capable of yielding mononuclear cell-containing plasma with little admixture of blood cell components other than mononuclear cells.

Using the composition for mononuclear cell-containing plasma separation according to the present invention, blood is collected and centrifuged in a blood sampling container containing the composition for mononuclear cell-containing plasma separation, whereby a partition is formed of the composition for mononuclear cell-containing plasma separation, and mononuclear cell-containing plasma is separated over the partition. The composition for mononuclear cell-containing plasma separation according to the present invention can easily and rapidly yield plasma containing mononuclear cells (lymphocytes and monocytes) from blood. In the present invention, mononuclear cells can be separated from blood by simple centrifugation, so that damage to mononuclear cells can be suppressed.

Using the composition for mononuclear cell-containing plasma separation according to the present invention, when the specific gravity at 25°C is within the range of 1.060 or more and 1.080 or less, the viscosity and thixotropic index of the composition for mononuclear cell-containing plasma separation can be improved. Accordingly, the flow of the composition for mononuclear cell-containing plasma separation can be suppressed during storage. In addition, even when the composition for mononuclear cell-containing plasma separation is contained in, for example, the bottom of a blood sampling main body, the composition for mononuclear cell-containing plasma separation can exhibit good fluidity and satisfactorily form a partition during centrifugation.

Using the composition for mononuclear cell-containing plasma separation according to the present invention, the obtained mononuclear cell-containing plasma can be used to analyze mononuclear cells with high accuracy.

The composition for mononuclear cell-containing plasma separation has a specific gravity of 1.060 or more and 1.080 or less at 25°C. When the specific gravity is less than 1.060 or more than 1.080, mononuclear cell-containing plasma cannot be satisfactorily separated from blood, the admixture of blood cell components other than mononuclear cells increases, and the mononuclear cell collection rate decreases.

The specific gravity of the composition for mononuclear cell-containing plasma separation at 25°C is preferably more than 1.060, more preferably 1.065 or more, and preferably less than 1.080, more preferably 1.077 or less, still more preferably 1.070 or less. When the specific gravity satisfies the above-mentioned preferable range, mononuclear cell-containing plasma can be satisfactorily separated from blood, and plasma with little admixture of blood cell components other than mononuclear cells and high content of mononuclear cells can be satisfactorily obtained. In particular, when the specific gravity is 1.065 or more, the mononuclear cell collection rate can be 70% or more. In addition, in particular, when the specific gravity is 1.070 or less, mononuclear cell-containing plasma in which the admixture of granulocytes is extremely small can be suitably obtained. For example, when the specific gravity is 1.070 or less, the granulocyte removal rate can be 90% or more. In addition, when the specific gravity satisfies the above-mentioned preferable range, a partition having good strength can be formed even at a low temperature or when the centrifugal force is low. Furthermore, when the specific gravity is equal to or higher than the above-mentioned lower limit, the mononuclear cell collection rate can be increased.

The specific gravity of the composition for mononuclear cell-containing plasma separation at 25°C is measured based on whether one drop of the composition for mononuclear cell-containing plasma separation sequentially added dropwise in saline solutions whose specific gravities are gradually adjusted at intervals of 0.002 at 25°C sinks or floats.

The viscosity of the composition for mononuclear cell-containing plasma separation at 25°C is preferably 100 Pa·s or more, more preferably 150 Pa·s or more, and preferably 400 Pa·s or less, more preferably 300 Pa·s or less. When the viscosity is the above-mentioned lower limit or more and the above-mentioned upper limit or less, the effect of the present invention can be exhibited even more effectively.

The viscosity of the composition for mononuclear cell-containing plasma separation at 25°C is measured under conditions at 25°C and a shear rate of 1.0 second⁻¹ using an E-type viscometer (for example, "TVE-35" manufactured by Toki Sangyo Co., Ltd.).

The thixotropic index of the composition for mononuclear cell-containing plasma separation at 25°C is preferably 1.1 or more, more preferably 1.2 or more, and preferably 1.5 or less, more preferably 1.3 or less. When the thixotropic index is the above-mentioned lower limit or more and the above-mentioned upper limit or less, the flow of the composition for mononuclear cell-containing plasma separation can be suppressed even more effectively during storage. In addition, even when the composition for mononuclear cell-containing plasma separation is contained in, for example, the bottom of a blood sampling main body, the composition for mononuclear cell-containing plasma separation can exhibit good fluidity and form a partition even more satisfactorily during centrifugation.

The thixotropic index of the composition for mononuclear cell-containing plasma separation at 25°C is calculated as follows. The viscosity value of the composition for mononuclear cell-containing plasma separation measured under conditions at 25°C and a shear rate of 1.0 second⁻¹ using an E-type viscometer is defined as a first viscosity value. The viscosity value of the composition for mononuclear cell-containing plasma separation measured under conditions at 25°C and a shear rate of 2.0 second⁻¹ using an E-type viscometer is defined as a second viscosity value. The ratio of the first viscosity value to the second viscosity value (first viscosity value/second viscosity value) is defined as a thixotropic index of the composition for mononuclear cell-containing plasma separation at 25°C. Examples of the E-type viscometer include "TVE-35" manufactured by Toki Sangyo Co., Ltd.

When mononuclear cell-containing plasma is separated from blood using the composition for mononuclear cell-containing plasma separation, the ratio of the number of erythrocytes contained in the separated mononuclear cell-containing plasma to the number of erythrocytes contained in the blood before separation (number of erythrocytes contained in the separated mononuclear cell-containing plasma/number of erythrocytes contained in blood before separation × 100) is defined as ratio (1). The ratio (1) is preferably 10% or less, more preferably 5% or less, still more preferably 1% or less.

When mononuclear cell-containing plasma is separated from blood using the composition for mononuclear cell-containing plasma separation, the ratio of the number of granulocytes contained in the separated mononuclear cell-containing plasma to the number of granulocytes contained in blood before separation (number of granulocytes contained in the separated mononuclear cell-containing plasma/number of granulocytes contained in blood before separation × 100) is defined as ratio (2). The ratio (2) is preferably 30% or less, more preferably 20% or less, still more preferably 10% or less.

When mononuclear cell-containing plasma is separated from blood using the composition for mononuclear cell-containing plasma separation, the ratio of the number of mononuclear cells contained in the separated mononuclear cell-containing plasma to the number of mononuclear cells contained in blood before separation (number of mononuclear cells contained in the separated mononuclear cell-containing plasma/number of mononuclear cells contained in blood before separation × 100) is defined as ratio (3). The ratio (3) is preferably 50% or more, more preferably 60% or more, still more preferably 70% or more.

Hereinafter, a detailed description is made of components contained in the composition for mononuclear cell-containing plasma separation according to the present invention.

### <Organic component having fluidity at 25°C>

The composition for mononuclear cell-containing plasma separation according to the present invention contains an organic component having fluidity at 25°C (hereinafter, sometimes abbreviated as "organic component"). The organic component may be used alone or in combination of two or more.

The above-mentioned "having fluidity at 25°C" means that the viscosity at 25°C is 500 Pa·s or less.

The viscosity of the organic component at 25°C is preferably 30 Pa·s or more, more preferably 50 Pa·s or more, and preferably 200 Pa·s or less, more preferably 100 Pa·s or less. When the viscosity is the above-mentioned lower limit or more and the above-mentioned upper limit or less, the fluidity of the organic component can be improved, the fluidity of the composition for mononuclear cell-containing plasma separation can be enhanced, and the strength of a partition can be enhanced.

The viscosity of the organic component at 25°C is measured under conditions at 25°C and a shear rate of 1.0 second⁻¹ using an E-type viscometer (for example, "TVE-35" manufactured by Toki Sangyo Co., Ltd.).

Examples of the organic component include a resin, and a mixture of a resin and an organic compound such as a plasticizer. When the organic component is a mixture of the resin and the organic compound, it is sufficient that the mixture (the organic component) itself have fluidity, and the resin or the organic compound may not have fluidity. When the organic component is a mixture of the resin and the organic compound, the resin may be, for example, a solid resin at 25°C. Each of the resin and the organic compound may be used alone or in combination of two or more.

From the viewpoint of exerting the effect of the present invention even more effectively, the organic component preferably contains the resin, more preferably a mixture of the resin and the organic compound. When the organic component contains two or more types of components (including the case where the organic component contains a solid component and a liquid component), these components may be mixed separately in a step of producing the composition for mononuclear cell-containing plasma separation.

Examples of the resin include a petroleum resin, a cyclopentadiene-based resin, a polyester resin, a polyurethane resin, a (meth)acrylic-based resin, a silicone resin, an α-olefin-fumaric acid ester copolymer, a copolymer of sebacic acid, 2,2-dimethyl-1,3-propanediol and 1,2-propanediol, a polyester polyurethane-based resin, and a polyether polyester-based resin. The resin may be used alone or in combination of two or more.

The petroleum resin includes a resin obtained by steam cracking of petroleum. Examples of the petroleum resin include a homopolymer or copolymer of a compound contained in the C₅ fraction (cyclopentadiene, isoprene, piperylene and 2-methylbutene-1,2-methylbutene-2, etc.); a homopolymer or copolymer of a compound contained in the C₉ fraction (styrene, vinyltoluene, α-methylstyrene, indene, coumarone, etc.); and a copolymer of a compound contained in the C₅ fraction and a compound contained in the C₉ fraction. The petroleum resin may be an unhydrogenated resin, a partially hydrogenated resin, or a completely hydrogenated resin.

Examples of commercially available products of the petroleum resin include "Regalite S5090" manufactured by Eastman Chemical Company.

Examples of the cyclopentadiene-based resin include a polymer of a cyclopentadiene-based monomer, a copolymer of a cyclopentadiene-based monomer and an aromatic monomer, and a dicyclopentadiene resin. The cyclopentadiene-based resin may be hydrogenated or otherwise. The polymer of a cyclopentadiene-based monomer, and the copolymer of a cyclopentadiene-based monomer and an aromatic monomer may be an oligomer.

Examples of the cyclopentadiene-based monomer include cyclopentadiene, dicyclopentadiene, and an alkyl-substituted derivative of cyclopentadiene.

Examples of the aromatic monomer include styrene, methylstyrene, indene, and methylindene.

Examples of commercially available products of the polymer of a cyclopentadiene-based monomer include "ESCOREZ5380", "ESCOREZ5300", "ESCOREZ5320", "ESCOREZ5340", "ESCOREZ5400", and "ESCOREZ ECR251" manufactured by Exxon Mobil.

Examples of commercially available products of the copolymer of a cyclopentadiene-based monomer and an aromatic monomer include "ESCOREZ ECR227E", "ESCOREZ ECR235E", "ESCOREZ ECR231C", "ESCOREZ5690", and "ESCOREZ5600" manufactured by Exxon Mobil.

Examples of commercially available products of the dicyclopentadiene resin include "SUKOREZ SU500" and "SUKOREZ SU90" manufactured by Colon.

Examples of the polyester resin include a polyalkylene terephthalate resin and a polyalkylene naphthalate resin. Examples of the polyalkylene terephthalate resin include polyethylene terephthalate, polybutylene terephthalate, and poly-1,4-cyclohexanedimethylene terephthalate.

Examples of the polyurethane resin include a reaction product of a polyol compound and an isocyanate compound.

The (meth)acrylic-based resin includes a resin obtained by polymerizing at least one (meth)acrylic ester monomer, and a resin obtained by polymerizing at least one (meth)acrylic ester monomer and a monomer other than the at least one (meth)acrylic ester monomer.

Examples of the (meth)acrylic ester monomer include an alkyl (meth)acrylic ester, a polyalkylene glycol (meth)acrylic ester, an alkoxyalkyl (meth)acrylic ester, a hydroxyalkyl (meth)acrylic ester, a glycidyl (meth)acrylic ester, a dialkylaminoalkyl (meth)acrylic ester, a benzyl (meth)acrylic ester, a phenoxyalkyl (meth)acrylic ester, a cyclohexyl (meth)acrylic ester, an isobornyl (meth)acrylic ester and an alkoxysilylalkyl (meth)acrylic ester having an alkyl group having 1 or more and 20 or less carbon atoms. The (meth)acrylic ester monomer may be used alone or in combination of two or more.

From the viewpoint of exerting the effect of the present invention even more effectively, the resin preferably includes a petroleum resin, a cyclopentadiene-based resin, a polyester resin or a polyurethane resin, more preferably includes a petroleum resin, a cyclopentadiene-based resin or a polyester resin, still more preferably includes a petroleum resin or a cyclopentadiene-based resin.

From the viewpoint of exerting the effect of the present invention even more effectively, the organic compound is preferably an alkyl benzenepolycarboxylate ester derivative. Accordingly, the organic component is preferably a mixture of the resin and the alkyl benzenepolycarboxylate ester derivative.

Examples of the alkyl benzenepolycarboxylate ester derivative include a phthalate ester, a trimellitate ester and a pyromellitate ester. The alkyl benzenepolycarboxylate ester derivative may be used alone or in combination of two or more.

Examples of the trimellitate ester include tri-n-octyl trimellitate, triisooctyl trimellitate and triisodecyl trimellitate.

Examples of the pyromellitate ester include tetraisooctyl pyromellitate.

Examples of commercially available products of the trimellitate ester include "MONOCIZER W700" and "MONOCIZER W-750" manufactured by DIC Corporation, "SANSO CIZER TOTM" and "SANSO CIZER TITM" manufactured by New Japan Chemical Co., Ltd.

Examples of commercially available products of the pyromellitate ester include "MONOCIZER W-7010" manufactured by DIC Corporation.

The alkyl benzenepolycarboxylate ester derivative is preferably a phthalate ester, a trimellitate ester, or a pyromellitate ester, more preferably a trimellitate ester.

The organic component also includes a liquid mixture of a poly-α-pinene polymer and a chlorinated hydrocarbon, a liquid mixture of a chlorinated polybutene and an epoxidized animal or vegetable oil, etc., a liquid mixture of trifluoroethylene chloride or an alkyl benzenepolycarboxylate ester derivative, etc. and polyoxyalkylene glycol, etc., and a mixture composed of a liquid/liquid or solid/liquid combination of a petroleum resin or a dicyclopentadiene resin, etc. and an alkyl benzenepolycarboxylate ester derivative, etc.

The specific gravity of the organic component at 25°C is preferably 1.020 or more, more preferably 1.030 or more, and preferably 1.080 or less, more preferably 1.060 or less, still more preferably 1.050 or less. When the specific gravity is the above-mentioned upper limit or less, the specific gravity of the composition for mononuclear cell-containing plasma separation does not become too high, and the partition can be satisfactorily formed. When the specific gravity is the above-mentioned lower limit or more, the specific gravity of the composition for mononuclear cell-containing plasma separation can be satisfactorily adjusted without adding a large amount of inorganic fine powder. When a large amount of inorganic fine powder is added, the fluidity of the composition for mononuclear cell-containing plasma separation is lowered, so that the partition cannot be satisfactorily formed.

The specific gravity of the organic component at 25°C is measured based on whether one drop of the organic component sequentially added dropwise in saline solutions whose specific gravities are gradually adjusted at intervals of 0.002 at 25°C sinks or floats.

### <Inorganic fine powder>

The composition for mononuclear cell-containing plasma separation according to the present invention contains two or more kinds of inorganic fine powders. As the inorganic fine powders, only 2 kinds may be used, or 3 or more kinds may be used.

Examples of the inorganic fine powders include a fine powder silica, a titanium oxide powder, a zinc oxide powder, an alumina powder, a glass fine powder, a talc powder, a kaolin powder, a bentonite powder, a titania powder, and a zirconium powder.

From the viewpoint of exerting the effect of the present invention more effectively, the inorganic fine powders preferably include a fine powder silica. In other words, it is preferable that one of two or more kinds of the inorganic fine powders be a fine powder silica. From the viewpoint of exerting the effect of the present invention even more effectively, the inorganic fine powders more preferably contain a fine powder silica and an inorganic fine powder different from the fine powder silica. The inorganic fine powder different from the fine powder silica is preferably an inorganic fine powder having a specific gravity of 3 or more. When adjusting the specific gravity of the composition for mononuclear cell-containing plasma separation to 1.060 or more and 1.080 or less using only a fine powder silica as the inorganic fine powders, it is necessary to blend a relatively large amount of fine powder silica, so that the viscosity and thixotropic index of the composition for mononuclear cell-containing plasma separation may become excessively high. Using a fine powder silica in combination with an inorganic fine powder that is different from the fine powder silica and has a specific gravity of 3 or more as the inorganic fine powders, it is possible to easily control the specific gravity of the composition for mononuclear cell-containing plasma separation while suitably maintaining the viscosity and the thixotropic index of the composition for mononuclear cell-containing plasma separation.

The fine powder silica includes a natural silica and a synthetic silica. The synthetic silica includes a hydrophilic silica and a hydrophobic silica. Since the quality is stable, the fine powder silica is preferably a synthetic silica, more preferably a synthetic silica produced by a vapor phase method. In the present invention, a fine powder silica is counted as one kind of inorganic fine powder.

A hydrophilic silica has the effect of imparting thixotropy to the composition for mononuclear cell-containing plasma separation and adjusting the specific gravity by hydrogen-bonding hydroxyl groups on the particle surface. On the other hand, a hydrophobic silica has a smaller effect of imparting thixotropy than that of a hydrophilic silica.

From the viewpoint of maintaining both the specific gravity and thixotropy of the composition for mononuclear cell-containing plasma separation within a suitable range, the fine powder silica preferably includes a hydrophilic silica, more preferably includes a hydrophilic silica and a hydrophobic silica. The fine powder silica preferably contains at least a hydrophilic silica.

The content of the hydrophilic silica in 100% by weight of the composition for mononuclear cell-containing plasma separation is preferably 0.01% by weight or more, more preferably 0.1% by weight or more, still more preferably 0.3% by weight or more, and preferably 2.50% by weight or less, more preferably 2.00% by weight or less. When the content of the hydrophilic silica is the above-mentioned lower limit or more and the above-mentioned upper limit or less, both the specific gravity and thixotropy of the composition for mononuclear cell-containing plasma separation can be maintained within a more suitable range. When the content of the above hydrophilic silica is more than 2.50% by weight, the viscosity and thixotropic index of the composition for mononuclear cell-containing plasma separation may become too high to satisfactorily form a partition during centrifugation, compared to when the content is 2.50% by weight or less.

The average particle diameter of the fine powder silica is not particularly limited. The average particle diameter of the fine powder silica may be 1 nm or more, 10 nm or more, 500 nm or less, or 100 nm or less.

The average particle diameters of the fine powder silica and the inorganic fine powder different from the fine powder silica described below are an average diameter measured on a volume basis, and are a median diameter value of 50% (D50). The volume average particle diameter (D50) can be measured by a laser diffraction/scattering method, an image analysis method, a Coulter method, a centrifugal sedimentation method, or the like. The volume average particle diameter (D50) is preferably determined by measurement using a laser diffraction/scattering method or an image analysis method.

Examples of commercially available products of the hydrophilic silica include AEROSIL (registered trademark) series such as AEROSIL 90G, 130, 200, 300, 200CF and 300CF (manufactured by Nippon Aerosil Co., Ltd.), REOLOSIL (registered trademark) series such as REOLOSIL QS-10, QS-20 and QS-30 (manufactured by Tokuyama Corporation), and WACKER HDK series such as WACKER HDK S13, N20 and T30 (manufactured by Wacker Asahikasei Silicone Co., Ltd.). The commercially available products of the hydrophilic silica are a hydrophilic silica produced by a vapor phase method, which is easy to use.

Examples of commercially available products of the hydrophobic silica include AEROSIL series such as Aerosil R972, R974, R805 and R812 (manufactured by Nippon Aerosil Co., Ltd.), REOLOSIL series such as REOLOSIL MT-10, DM-30S, HM-30S, KS-20S and PM-20 (manufactured by Tokuyama Corporation), and WACKER HDK series such as WACKER HDK H15, H18 and H30 (manufactured by Wacker Asahikasei Silicone Co., Ltd.). The commercially available products of the hydrophobic silica are a hydrophobic silica produced by a vapor phase method, which is easy to use.

Examples of the inorganic fine powder that is different from the fine powder silica and has a specific gravity of 3 or more include a titanium oxide powder, a zinc oxide powder and an alumina powder.

From the viewpoint of maintaining the thixotropy and specific gravity within a suitable range, the inorganic fine powder having a specific gravity of 3 or more is preferably a titanium oxide powder or a zinc oxide powder.

The specific gravity of the inorganic fine powder having a specific gravity of 3 or more is preferably 3.5 or more, more preferably 4 or more. The larger the specific gravity of the inorganic fine powder having a specific gravity of 3 or more, the better. When the specific gravity is the above-mentioned lower limit or more, the specific gravity of the composition for mononuclear cell-containing plasma separation can be effectively increased.

The average particle diameter of the inorganic fine powder having a specific gravity of 3 or more is not particularly limited. The average particle diameter of the inorganic fine powder having a specific gravity of 3 or more may be 10 nm or more, 100 nm or more, 10 µm or less, or 1 µm or less.

The content of the inorganic fine powder having a specific gravity of 3 or more in 100% by weight of the composition for mononuclear cell-containing plasma separation is preferably 0.01% by weight or more, more preferably 0.1% by weight or more, still more preferably 1% by weight or more, and preferably 10% by weight or less, more preferably 5% by weight or less. When the content of the inorganic fine powder having a specific gravity of 3 or more is the above-mentioned lower limit or more and the above-mentioned upper limit or less, the effect of the present invention can be exhibited even more effectively. In addition, when the content of the inorganic fine powder having a specific gravity of 3 or more is the above-mentioned lower limit or more and the above-mentioned upper limit or less, the specific gravity of the composition for mononuclear cell-containing plasma separation can be effectively increased.

### <Other components>

The composition for mononuclear cell-containing plasma separation according to the present invention may contain components other than the above-mentioned component, as long as the effect of the present invention is not impaired. The composition for mononuclear cell-containing plasma separation of the present invention may, for example, contain as the other components an organic gelling agent, a thermoplastic elastomer, a polyalkylene glycol, a silicone oil, an auxiliary solvent, an antioxidant, a colorant, and water. The respective other components may be used alone or in combination of two or more.

Examples of the organic gelling agent include dibenzylidene sorbitol, a dibenzylidene sorbitol derivative and a fatty acid amide. By containing the organic gelling agent, the thixotropy can be further improved.

Examples of the commercially available products of the dibenzylidene sorbitol and the dibenzylidene sorbitol derivative include "GEL ALL MD" and "GEL ALL D" manufactured by New Japan Chemical Co., Ltd.

Examples of the thermoplastic elastomer include a styrene-butadiene-styrene copolymer (SBS), a styrene-isoprene-styrene copolymer (SIS), a styrene-ethylene-butylene-styrene copolymer (SEBS), a styrene-butadiene-butylene-styrene copolymer (SBBS), a styrene-ethylene-propylene-styrene copolymer (SEPS), a styrene-butadiene copolymer (SB), a styrene-isoprene copolymer (SI), a styrene-ethylene-butylene copolymer (SEB), a styrene-butadiene-butylene copolymer (SBB) and a styrene-ethylene-propylene copolymer (SEP), and a modified product thereof.

Examples of the polyalkylene glycol include polybutylene glycol, polypropylene glycol, polyoxypropylene glyceryl ether, polyoxypropylene sorbitol, polyserine, polyoxypropylene diglyceryl ether, polyoxyethylene polyoxypropylene glycol, polyoxyethylene polyoxypropylene glyceryl ether, polyoxypropylene butyl ether, polyoxypropylene glycol monoether and polyoxypropylene alkyl ether, and modified products thereof.

Examples of the silicone oil include a dimethyl silicone oil, a methyl phenyl silicone oil, a methyl hydrogen silicone oil, an alkyl-modified silicone oil, an aralkyl-modified silicone oil, a fluorine-modified silicone oil, a polyether-modified silicone oil, an amino-modified silicone oil, an epoxy-modified silicone oil, a phenol-modified silicone oil, a carboxy-modified silicone oil, a methacrylate-modified silicone oil, and an alkoxy-modified silicone oil.

Examples of the auxiliary solvent include toluene, N,N-dimethylformamide, 1-methyl-2-pyrrolidone, and dimethyl sulfoxide. By using the auxiliary solvent, for example, an organic gelling agent, a thermoplastic elastomer and the like can be easily blended.

The composition for mononuclear cell-containing plasma separation may contain water or otherwise. The water is preferably purified water. The lower the water content, the better.

### <Method for producing composition for mononuclear cell-containing plasma separation>

The method for producing the composition for mononuclear cell-containing plasma separation is not particularly limited. The composition for mononuclear cell-containing plasma separation can be produced, for example, by mixing the organic component having fluidity at 25°C, the inorganic fine powders, and the other component to be blended as needed. The mixing method is not particularly limited, but the mixture may be mixed by a publicly known kneader such as a planetary mixer, a ball mill, or a disper.

### <Blood sampling container>

The blood sampling container according to the present invention includes a blood sampling container main body and the composition for mononuclear cell-containing plasma separation, the composition for mononuclear cell-containing plasma separation being contained in the blood sampling container main body.

The composition for mononuclear cell-containing plasma separation is preferably contained in the bottom of the blood sampling container main body. In the present invention, even when the composition for mononuclear cell-containing plasma separation is contained in the bottom of the blood sampling main body, the partition can be satisfactorily formed during centrifugation.

The material for the blood sampling container main body is not particularly limited, but examples thereof include publicly known materials such as a thermoplastic resin such as polyethylene (PE), polypropylene (PP), polystyrene (PS), polyethylene terephthalate (PET), polymethylmethacrylate, polyacrylonitrile, polyamide, an acrylonitrile-styrene copolymer or an ethylene-vinyl alcohol copolymer; a thermosetting resin such as an unsaturated polyester resin, an epoxy resin or an epoxy-acrylate resin; a modified natural resin such as cellulose acetate, cellulose propionate, ethyl cellulose or ethyl chitin; silicate such as soda-lime glass, phosphosilicate glass or borosilicate glass, glass such as quartz glass, a combination of them, or one containing them as the main components.

The blood sampling container main body may be sealed with a stopper or a sealing member such as an aluminum seal.

The internal pressure in the blood sampling container is not particularly limited. The blood sampling container can also be used as a vacuum blood sampling tube that has been sealed with the sealing member after the gas is discharged from the inside. In the case of a vacuum blood sampling tube, a certain amount of blood can be easily collected regardless of the technical difference between blood collectors.

From the viewpoint of preventing bacterial infection, the inside of the blood sampling container is preferably sterilized in accordance with the ISO and JIS standards.

A publicly known drug such as a blood adhesion inhibitor may be attached to the inner wall of the blood sampling container main body, depending on the purpose of preventing blood adhesion, etc.

An anticoagulant is preferably contained in the blood sampling container main body. In this case, the anticoagulant may be attached to the inner wall of the blood sampling container main body. The anticoagulant may be added to collected blood. Examples of the anticoagulant include heparin, ethylenediaminetetraacetic acid (EDTA) and citric acid.

### <Method for separating mononuclear cell-containing plasma>

The composition for mononuclear cell-containing plasma separation according to the present invention can be used to separate mononuclear cell-containing plasma from blood. The method for separating mononuclear cell-containing plasma preferably includes a step of collecting blood in the blood sampling container main body (blood collection step) and a step of centrifuging the blood sampling container in which blood has been collected (centrifugation step).

In the blood collection step, it is preferable that the anticoagulant be contained in the blood sampling container main body, or blood to which the anticoagulant has been added be collected in the blood sampling container main body.

A centrifugation condition in the centrifugation step is not particularly limited as long as a partition can be formed of the composition for mononuclear cell-containing plasma separation to separate mononuclear cells and blood cell components other than the mononuclear cells. Examples of the centrifugation condition include a condition for centrifugation at 400 G or more and 4000 G or less for 10 minutes or more and 120 minutes or less. In the centrifugation step, erythrocytes and granulocytes are located under the partition formed of the composition for mononuclear cell-containing plasma separation, and mononuclear cell-containing plasma is located thereover.

Hereinafter, more detailed description is made of the present invention with reference to Examples. The present invention is not limited to the following Examples only.

The followings were prepared as materials for the composition for mononuclear cell-containing plasma separation.

### (Material for organic component having fluidity at 25°C)

Resin:
   Petroleum resin ("Regalite S5090" manufactured by Eastman Chemical Company)
   Dicyclopentadiene resin 1 ("SUKOREZ SU500" manufactured by Colon)
   Dicyclopentadiene resin 2 ("SUKOREZ SU90" manufactured by Colon)
Organic compound:
   Trimellitate ester (alkyl benzenepolycarboxylate ester derivative, "MONOCIZER W700" manufactured by DIC)

### (Inorganic fine powder)

Hydrophilic silica (fine powder silica, "200CF" manufactured by Nippon Aerosil Co., Ltd., specific gravity 2.2)
Hydrophobic silica (fine powder silica, "R974" manufactured by Nippon Aerosil Co., Ltd., specific gravity 2.2)
Titanium oxide powder ("A-100" manufactured by Ishihara Sangyo Kaisha, Ltd., specific gravity 4)
Zinc oxide powder ("Zncox Super F-2" manufactured by Hakusui Tech, specific gravity 5.6)

### (Other components)

Organic gelling agent ("GEL ALL D" manufactured by New Japan Chemical Co., Ltd.)
1-Methyl-2-pyrrolidone (auxiliary solvent)
Thermoplastic Elastomer ("3421C" manufactured by Zeon Corporation)

### (Example 1)

Preparation of organic component having fluidity at 25°C:
The materials for organic component having fluidity at 25°C shown in Table 1 were blended, heated and dissolved at 130°C, and mixed to prepare an organic component having fluidity at 25°C.

Preparation of composition for mononuclear cell-containing plasma separation:
A composition for mononuclear cell-containing plasma separation was prepared by mixing the organic component having fluidity at 25°C, the inorganic fine powders, and the other components at the blending ratios shown in Table 1.

### (Examples 2 to 5 and Comparative Examples 1 to 3)

Compositions for mononuclear cell-containing plasma separation were prepared in the same manner as in Example 1 except that the types and blending amounts of the compounding components were modified as shown in Table 1.

### (Evaluation)

### (1) Specific gravity

The specific gravity was measured based on whether one drop of the obtained organic component having fluidity at 25°C or one drop of the obtained composition for mononuclear cell-containing plasma separation sequentially added dropwise in saline solutions whose specific gravities were gradually adjusted at intervals of 0.002 at 25°C sank or floated.

### (2) Viscosity

The viscosity of the obtained composition for mononuclear cell-containing plasma separation at 25°C was measured under conditions at 25°C and a shear rate of 1.0 second⁻¹ using an E-type viscometer ("TVE-35" manufactured by Toki Sangyo Co., Ltd.).

### (3) Thixotropic index

A first viscosity value was determined by measuring the obtained composition for mononuclear cell-containing plasma separation under conditions at 25°C and a shear rate of 1.0 second⁻¹ using an E-type viscometer ("TVE-35" manufactured by Toki Sangyo Co., Ltd.). Similarly, a second viscosity value was determined by measuring the obtained composition for mononuclear cell-containing plasma separation under conditions at 25°C and a shear rate of 2.0 second⁻¹ using an E-type viscometer ("TVE-35" manufactured by Toki Sangyo Co., Ltd.). The ratio of the first viscosity value to the second viscosity value (first viscosity value/second viscosity value) was calculated as a thixotropic index of the composition for mononuclear cell-containing plasma separation at 25°C.

### (4) Flow

A blood sampling container was prepared by placing 1.0 g of the obtained composition for mononuclear cell-containing plasma separation in the bottom of a transparent PET bottomed tube (blood sampling container main body) having an inner diameter of 14 mm and a length of 100 mm, followed by plugging with a butyl rubber stopper. Next, the blood sampling container was tilted such that the open end side of the blood sampling container main body faced diagonally downward by 45° and the bottom side thereof faced diagonally upward by 45°. In this state, the blood sampling container was allowed to stand in an oven kept at 55°C for 1 day. The distance that the composition for mononuclear cell-containing plasma separation had flowed downward along the inner wall of the blood sampling container from before to after standing was measured using a caliper.

### (5) Formability of partition

Preparation of blood sampling container:
A PET bottomed tube (blood sampling container main body) having a length of 100 mm and an inner diameter of the opening of 14 mm was prepared. In the bottom of the blood sampling container main body was placed 1.0 g of the obtained composition for mononuclear cell-containing plasma separation. In addition, ethylenediaminetetraacetic acid (EDTA) was attached to the inner wall of the blood sampling container main body, and the pressure inside the blood sampling container was reduced to 50 kPa, followed by sealing with a butyl rubber stopper. In this way, a blood sampling container with the composition for mononuclear cell-containing plasma separation contained in the blood sampling container main body was prepared.

Blood was prepared from 3 people, and the following steps were performed in this order.

Blood collection step:
In the blood sampling container main body of the obtained blood sampling container was collected 4 mL of blood.

Centrifugation step:
The blood sampling container was centrifuged at 1500 G for 30 minutes.

Visual observation:
After centrifugation, it was visually confirmed whether or not the composition for mononuclear cell-containing plasma separation contained in the bottom of the blood sampling container main body prior to centrifugation moved between the erythrocyte layer and the plasma layer to satisfactorily form a partition. Specifically, the formability of the partition was evaluated by the following method.

Test (1): The blood sampling container after centrifugation was visually observed. When the composition for mononuclear cell-containing plasma separation was located between the erythrocyte layer and the plasma layer, it was judged to be good. On the other hand, when the total amount of the composition for mononuclear cell-containing plasma separation was located under the erythrocyte layer, it was judged to be poor.

Test (2): The blood sampling container after centrifugation was allowed to stand such that the bottom of the blood sampling container faced upward by 90°. At this time, it was visually confirmed whether or not the blood cell component located nearer to the bottom side of the blood sampling container than the composition for mononuclear cell-containing plasma separation moved nearer to the open end side of the blood sampling container than the composition for mononuclear cell-containing plasma separation to mix with plasma. When the blood cell component did not mix with plasma, it was judged to be good. On the other hand, when the blood cell component mixed with plasma, it was judged to be poor.

### [Criteria for formability of partition]

∘: The judgment results of both test (1) and test (2) are good.
×: The judgment result of either test (1) or test (2) is poor.

### (6) Collection of mononuclear cell-containing plasma

After the centrifugation step in "(5) Formability of partition", a blood cell component located over the partition formed of the composition for mononuclear cell-containing plasma separation was suspended in plasma located above the blood cell component, and the plasma containing the blood cell component was collected. In Comparative Example 3, since the partition was not formed, this evaluation was not performed.

The number of cells for blood cell components (erythrocytes, granulocytes (neutrophils, eosinophils and basophils), mononuclear cells (lymphocytes and monocytes)) in the separated plasma was measured by analyzing the collected solution using a multi-item automatic blood cell analyzer ("XE5000" manufactured by Sysmex Corporation). Also for prepared blood (whole blood sample), similarly, the number of cells for blood cell components (erythrocytes, granulocytes (neutrophils, eosinophils and basophils), mononuclear cells (lymphocytes and monocytes)) in the whole blood sample was measured. The concentration of blood cell components is an average value of the results obtained by evaluating the individual prepared bloods from the 3 people.

The erythrocyte removal rate, granulocyte removal rate, and mononuclear cell collection rate were individually calculated by the following formulas.

Erythrocyte removal rate (%) = 100 - [(number of erythrocytes contained in separated plasma)/(number of erythrocytes contained in whole blood sample) × 100]

Granulocyte removal rate (%) = 100 - [(number of granulocytes contained in separated plasma)/(number of granulocytes contained in whole blood sample) × 100]

Mononuclear cell collection rate (%) = (number of mononuclear cells contained in separated plasma)/(number of mononuclear cells contained in whole blood sample) × 100

The compositions and results are shown in Table 1 below.

**[Table 1]**

| | | | | | | Example 1 | Example 2 | Example 3 | Example 4 | Example 5 | Comparative Example 1 | Comparative Example 2 | Comparative Example 3 | Whole blood sample |
|---|---|---|---|---|---|---|---|---|---|---|---|---|---|---|
| Organic component having fluidity at 25°C | Petroleum resin | | | | % by weight | 14.17 | 14.11 | 13.99 | - | 13.91 | 14.50 | 13.75 | 13.92 | - |
| | Dicyclopentadiene resin 1 | | | | % by weight | 17.62 | 17.54 | 17.40 | - | 17.29 | 18.03 | 17.09 | 17.31 | - |
| | Dicyclopentadiene resin 2 | | | | % by weight | 19.29 | 19.21 | 19.05 | 50.95 | 18.93 | 19.74 | 18.71 | 18.95 | - |
| | Trimellitate ester | | | | % by weight | 43.77 | 43.58 | 43.20 | 41.89 | 42.91 | 44.87 | 42.39 | 42.96 | - |
| Inorganic fine powder | Fine powder silica | Hydrophilic silica | | | % by weight | 0.71 | 0.71 | 0.71 | 1.00 | 0.71 | 0.71 | 0.71 | 0.71 | - |
| | | Hydrophobic silica | | | % by weight | 1.79 | 1.79 | 1.79 | 1.50 | 1.79 | 1.79 | 1.79 | 5.79 | - |
| | Titanium oxide powder | | | | % by weight | 2.30 | 2.70 | 3.50 | - | 4.10 | - | 5.20 | - | - |
| | Zinc oxide powder | | | | % by weight | - | - | - | 2.60 | - | - | - | - | - |
| Other components | Organic gelling agent | | | | % by weight | 0.06 | 0.06 | 0.06 | 0.06 | 0.06 | 0.06 | 0.06 | 0.06 | - |
| | 1-Methyl-2-pyrrolidone | | | | % by weight | 0.30 | 0.30 | 0.30 | - | 0.30 | 0.30 | 0.30 | 0.30 | - |
| | Thermoplastic elastomer | | | | % by weight | - | - | - | 2.00 | - | - | - | - | - |
| Total | | | | | % by weight | 100 | 100 | 100 | 100 | 100 | 100 | 100 | 100 | - |
| Organic component having fluidity at 25°C | | | Specific gravity at 25°C | | - | 1.033 | 1.033 | 1.033 | 1.044 | 1.033 | 1.033 | 1.033 | 1.033 | - |
| Composition for mononuclear cell-containing plasma separation | | | Viscosity at 25°C | | Pa•s | 176 | 172 | 179 | 286 | 180 | 175 | 197 | 856 | - |
| | | | Thixotropic index at 25°C | | - | 1.28 | 1.26 | 1.27 | 1.27 | 1.26 | 1.25 | 1.29 | 1.54 | - |
| | | | Specific gravity at 25°C | | - | 1.063 | 1.065 | 1.070 | 1.073 | 1.077 | 1.045 | 1.085 | 1.068 | - |
| Flow | | | | | mm | 0.5 | 0.6 | 0.5 | 0.4 | 0.7 | 1 | 0.2 | 0.3 | - |
| Formability of partition | | | | | - | ∘ | ∘ | ∘ | ∘ | ∘ | ∘ | ∘ | × | - |
| Collection of mononuclear cell-containing plasma | Number of blood cell components in separated plasma or whole blood sample | Erythrocyte | | | cells | 4450000 | 56000000 | 55000000 | 560000 | 66550000 | 40000000 | 520000000 | - | 17550000000 |
| | | Leukocyte | Granulocyte | | cells | 451000 | 580000 | 710000 | 1600000 | 2100000 | 56000 | 10500000 | - | 11900000 |
| | | | Mononuclear cell | Lymphocyte | cells | 4200000 | 4800000 | 5050000 | 5250000 | 5800000 | 1420000 | 6500000 | - | 6550000 |
| | | | | Monocyte | cells | 510000 | 650000 | 755000 | 740000 | 730000 | 120000 | 1130000 | - | 1200000 |
| | | | | Total | cells | 5161000 | 6030000 | 6515000 | 7590000 | 8630000 | 1596000 | 18130000 | - | 19650000 |
| | Erythrocyte removal rate | | | | % | 100 | 100 | 100 | 100 | 100 | 100 | 97 | - | - |
| | Granulocyte removal rate | | | | % | 96 | 95 | 94 | 87 | 82 | 100 | 12 | - | - |
| | Mononuclear cell collection rate | | | | % | 61 | 70 | 75 | 77 | 84 | 20 | 98 | - | - |

It can be found that the compositions for mononuclear cell-containing plasma separation having a specific gravity of 1.060 or more and 1.080 or less obtained in Examples 1 to 5 result in a high mononuclear cell collection rate, and high erythrocyte and granulocyte removal rates. On the other hand, the composition for mononuclear cell-containing plasma separation having a specific gravity of less than 1.060 obtained in Comparative Example 1 can result in high erythrocyte and granulocyte removal rates, but results in a low mononuclear cell collection rate. In addition, the composition for mononuclear cell-containing plasma separation having a specific gravity of more than 1.080 obtained in Comparative Example 2 can result in a high mononuclear cell collection rate, but results in low erythrocyte and granulocyte removal rates.

In particular, it can be found that the compositions for mononuclear cell-containing plasma separation having a specific gravity of 1.060 or more and 1.070 or less obtained in Examples 1 to 3 can result in a particular high granulocyte removal rate.

In addition, the compositions for mononuclear cell-containing plasma separation containing two or more kinds of inorganic fine powders (a fine powder silica and an inorganic fine powder having a specific gravity of 3 or more) obtained in Examples 1 to 5 can improve the viscosity and thixotropic index of the composition for mononuclear cell-containing plasma separation, can suppress the flow of the composition for mononuclear cell-containing plasma separation during storage, and can satisfactorily form a partition during centrifugation. On the other hand, the composition for mononuclear cell-containing plasma separation obtained in Comparative Example 3 in which only a fine powder silica (one kind of inorganic fine powder) is used as the inorganic fine powder can effectively suppress the flow of the composition for mononuclear cell-containing plasma separation during storage, but not sufficiently flow during centrifugation to form a partition.

## Claims

1. A composition for mononuclear cell-containing plasma separation,
comprising an organic component having fluidity at 25°C and two or more kinds of inorganic fine powders, and
having a specific gravity of 1.060 or more and 1.080 or less at 25°C.

2. The composition for mononuclear cell-containing plasma separation according to claim 1, wherein the organic component having fluidity at 25°C contains a resin.

3. The composition for mononuclear cell-containing plasma separation according to claim 2, wherein the organic component having fluidity at 25°C is a mixture of the resin and an alkyl benzenepolycarboxylate ester derivative.

4. The composition for mononuclear cell-containing plasma separation according to claim 2 or 3, wherein the resin includes a petroleum resin, a cyclopentadiene-based resin, or a polyester resin.

5. The composition for mononuclear cell-containing plasma separation according to any one of claims 2 to 4, wherein the resin includes a petroleum resin or a cyclopentadiene-based resin.

6. The composition for mononuclear cell-containing plasma separation according to any one of claims 1 to 5, wherein the inorganic fine powders include a fine powder silica.

7. The composition for mononuclear cell-containing plasma separation according to any one of claims 1 to 6, wherein the inorganic fine powders include a fine powder silica and an inorganic fine powder having a specific gravity of 3 or more.

8. The composition for mononuclear cell-containing plasma separation according to claim 6 or 7, wherein the fine powder silica includes a hydrophilic silica.

9. The composition for mononuclear cell-containing plasma separation according to any one of claims 6 to 8, wherein the fine powder silica includes a hydrophilic silica and a hydrophobic silica.

10. The composition for mononuclear cell-containing plasma separation according to any one of claims 1 to 9, comprising an organic gelling agent.

11. A blood sampling container, comprising
a blood sampling container main body and
the composition for mononuclear cell-containing plasma separation according to any one of claims 1 to 10,
the composition for mononuclear cell-containing plasma separation being contained in the blood sampling container main body.
